# EUROPEAN PATENT SPECIFICATION

(11) **EP 1 563 811 B1**
(45) Date of publication and mention of the grant of the patent: **24.08.2011**
(21) Application number: 04105833.0
(22) Date of filing: 17.11.2004
(51) Int. Cl.: A61F 5/01

(54) **Orthosis for the protection of the wrist, particularly for use IN SPORTS**
Orthese zum Schutze des Handgelenkes, vor allem zur Sportausübung.
Orthèse pour la protection du poignet, notament pour la pratique sportive.

(30) Priority: 17.12.2003 IT RM20030579
(43) Date of publication of application: 17.08.2005
(73) Proprietor: Dainese S.p.A., 36060 Molvena (IT)
(72) Inventor: ORLANDO, Oliviero, 35031, Abano Terme PD (IT)
(74) Representative: Manfrin, Marta

(56) References cited:
- EP-A1- 0 631 735
- WO-A-01/60289
- FR-A- 2 650 176
- FR-A- 2 686 246
- US-A- 6 029 277
- US-A1- 2003 163 075
- US-B1- 6 475 174

## Description

The present invention relates to an orthosis for the protection of the wrist, particularly for use in sports, of the kind comprising stiff or semiflexible protection elements, anyhow at least partially capable of absorbing a shock, arranged at the wrist joint.

The present invention further relates to a glove or a wrist brace incorporated in said orthosis.

There are several known protections for protecting the wrist during sports activities entailing falls, even at high speed, like snowboard, ski or rollerblade practice. The purpose of these protections is to prevent shocks in the wrist region, but above all to prevent shocks at the wrist: sudden rotations in unnatural directions causing extensions or tearing of ligaments, sprains and fractures.

As an example, EP 0631735 discloses a wrist guard for protecting a wearer's wrist, including a first rigid plate member and a second rigid plate member; the plate members are connected by a hinge such that the first and second plate members pivot relative to each other about an axis generally parallel to a bending axis of the wrist.

Among known protections there are the stiff and non-anatomical ones, hampering any motion and thereby hindering sports practice and proving umconfortable. Also protections with stiff splints longitudinally arranged at a glove in the wrist region are known. However, such a protection blocks the entire forearm and the hand. Lastly, palm protections restricting sideway torsions of the wrist are known, being however insufficient for complete protection.

The technical problem underlying the present invention is to provide an orthosis for the protection of the wrist overcoming the drawbacks mentioned with reference to the known art.

Such a problem is solved by an orthosis as abovespecified, comprising:
* means for wearing the orthosis on the wrist region, comprising a portion of the back of the hand, the wrist joint and a portion of forearm adjacent to the wrist;
* at least one longitudinal protection and stiffening element, associated to said means for wearing the orthosis on so as to extend over the entire wrist region;
* a guide longitudinally arranged at said at least one longitudinal protection element, astride of the wrist joint;
* a shield-shaped protection element slidably associated to said guide; and
* means for fastening said shield-shaped protection element in a desired position.

The main advantage of the orthosis according to the invention is to allow the correct positioning of a protection element capable of absorbing shocks, in a correct position to prevent wrist shocks, concomitantly allowing a greater freedom of motion to the wrist.

The present invention will hereinafter be described according to two preferred embodiments thereof, given by way of example and not for limitative purposes, with reference to the annexed drawings, wherein:
* Figure 1 shows a partially exploded perspective top view of a first embodiment of an orthosis for the protection of the wrist according to the invention;
* Figure 2 shows a perspective top view of the orthosis of Figure 1;
* Figure 3 shows a perspective bottom or upturned view of the orthosis of Figure 1;
* Figure 4 shows a longitudinal sectional view of a detail of the orthosis of Figure 1, taken along line A-A of Figure 2;
* Figure 5 shows a perspective top view of the orthosis of Figure 1 worn on a left hand;
* Figure 6 shows a perspective top view of a second embodiment of an orthosis for the protection of the wrist according to the invention, worn on a left hand; and
* Figure 7 shows a side elevational view of the orthosis of the preceding figure.

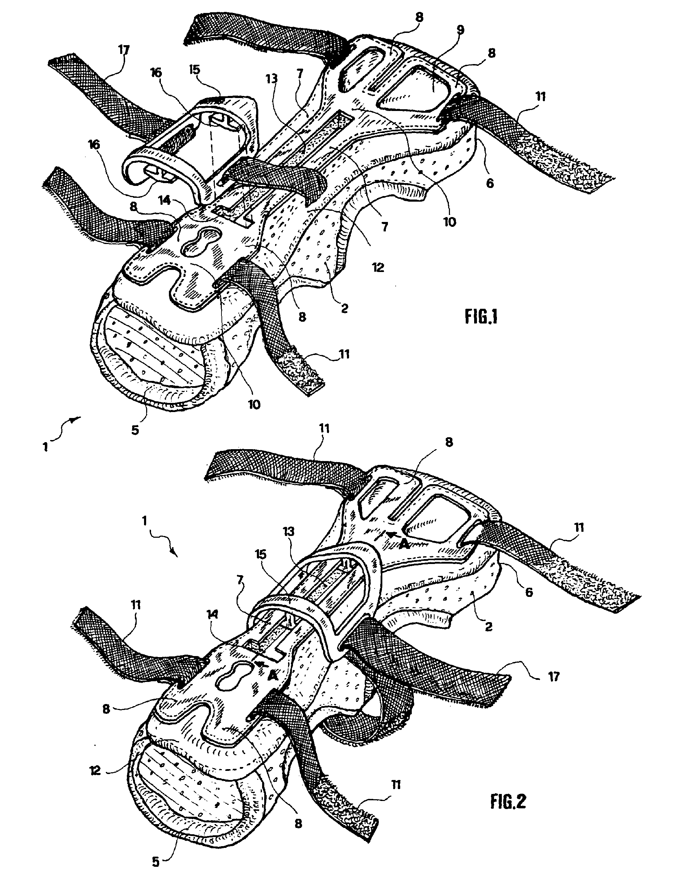

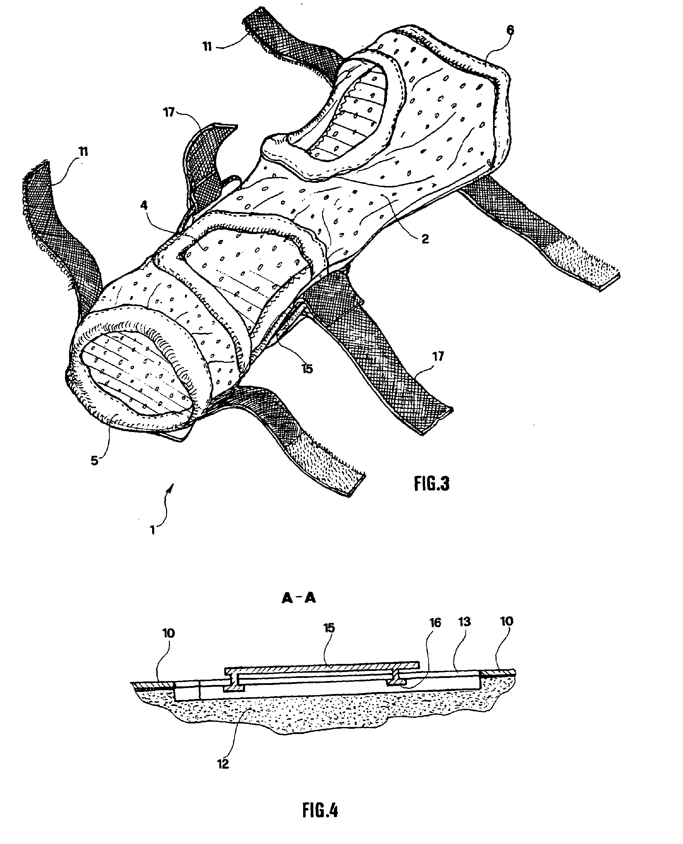

With reference to Figures 1 to 5, hereinafter there will be described a first embodiment of orthosis for the protection of the wrist, generally indicated by 1. A left-hand design is depicted, yet it is understood that the same invention relates to an analogous and specular right-hand design.

The orthosis 1 comprises means for wearing the orthosis on the wrist region, the latter being hereinafter defined as comprising a portion of the back of the hand, the wrist joint and a portion of forearm adjacent to the wrist, i.e., the back region of the end of an arm.

In the present embodiment said means for wearing on comprises a wrist brace 2, sleeve-shaped but having a first port 3 for the thumb and a second port 4 at the bottom fleshy part of the wrist. Such a wrist brace 2 is preferably made of breathable fabric. It comprises a proximal end 5, at the forearm, and a distal end 6, at the hand. Its back portion is void of ports, and two longitudinal protection and stiffening elements 7, extending over the entire wrist region, are associated thereon, by edge seaming or analogous systems.

These longitudinal protection elements 7, which in modified versions can be one or three or more in number, operate substantially as splints, yet are fixed to the wrist brace 2 so as to be unmovable. The former are made of a stiff or semiflexible material, capable of resisting shocks and of absorbing them, yet elastic enough not to completely hamper wrist motion.

In the embodiments described hereto, they are made by cutting or die cutting a plane element of a suitable material, implementing two elongate elements partitioned by a central slit and each having notches at their ends.

Therefore, said elements 7 have, onto their ends, widened portions 8 provided with notches 9 to limit stiffness and allow perspiration. Moreover, the elements 7 are united by longitudinal junctions 10, so as to allow the relative rotation of one element 7 with respect to the other one, and vice versa.

At said widened portions 8, the orthosis 2 comprises first means for fastening, which in the present embodiment is implemented by straps 11 provided with Velcro® at their ends in order to adjust its tensioning. The Velcro® may be replaced by analogous tensioning adjustment systems.

Between the wrist brace 2 and the longitudinal protection elements 7 there is arranged an absorbing layer 12, of flexible material and certain thickness, having the purpose of absorbing shocks. This absorbing layer is of a shape such as to cover substantially the entire wrist region.

Between said longitudinal protection elements 7 there is located a guide 13, in the form of a groove, arranged longitudinally astride of the wrist joint. In the present embodiment such a guide 13 is obtained between the longitudinal junctions 10 and it is delimited by the same elements 7. The guide 13, at one end thereof, has a transverse through recess 14.

The orthosis 1 comprises a shield-shaped protection element 15 that is slidably associated to said guide 13. Said element 15 is substantially shaped like a quadrilateral frame with slightly curved sides, in order to extend and entirely cover the wrist joint.

Onto its bottom face, the shield-shaped protection element 15 comprises a pair of projections 16 for sliding, shaped like an upturned T so as to be capable of going through said transverse recess 14 and inserting below the guide 13, with the stem of each projection 16 going through the port of the guide 13.

In addition, the orthosis 1 comprises second means for fastening said shield-shaped protection element in a desired position. Said second means, in the present embodiment, is implemented by straps 17 provided with Velcro® at their ends in order to adjust its tensioning. The Velcro® may be replaced by analogous tensioning adjustment systems.

It is proposed that the orthosis 1 may be normally worn on the wrist, by positioning said longitudinal protection elements 7 and said absorbing layer 12 on the back. The orthosis 1 may then be secured by virtue of the first means for fastening. Subsequently, the shield-shaped element 15 may be positioned exactly at the wrist joint, a position that obviously varies according to anatomy.

Thus, on the hand an optimal protection from shocks is ensured, and, on the other hand, an element such as to prevent wrist shocks and yet of dimensions not hampering a wrist in its motions is exactly positioned. Moreover, during natural wrist motions, the shield-shaped element 15 may slide imperceptibly, increasing the freedom of motion of the wrist.

On the contrary, in case of shock to the wrist, the shield-shaped element is stuck in a correct position by the deformation of the guide 13; thus, the element 15, favoured by its being in a position ever more raised with respect to the wrist brace 2, effectively restricts an unnatural backward rotation of the wrist.

Thus, the shocks gradually discharge on a surface greater with respect to that of the point of impact. The restriction to the sudden rotation of the wrist also prevents the breaking of the joint, of the navicular bone and the tearing of the ligaments associated thereto.

Lastly, an anatomically designed and dynamic configuration contouring the protected limb, and a light-weight and breathable protection are attained.

Moreover, this embodiment is susceptible of being covered by a handle for the protection from snow, of the kind commonly used in snowboard practice.

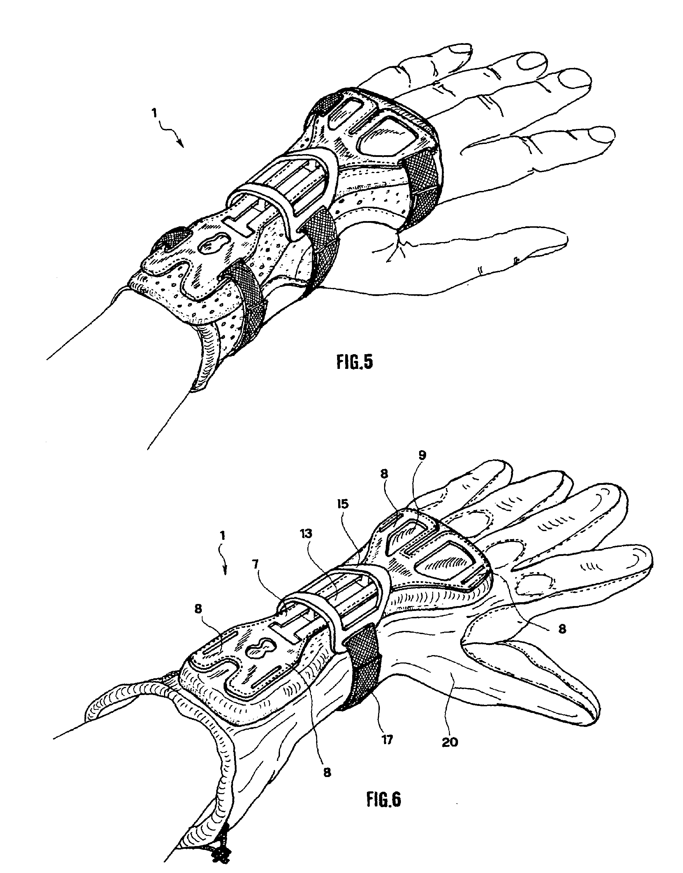

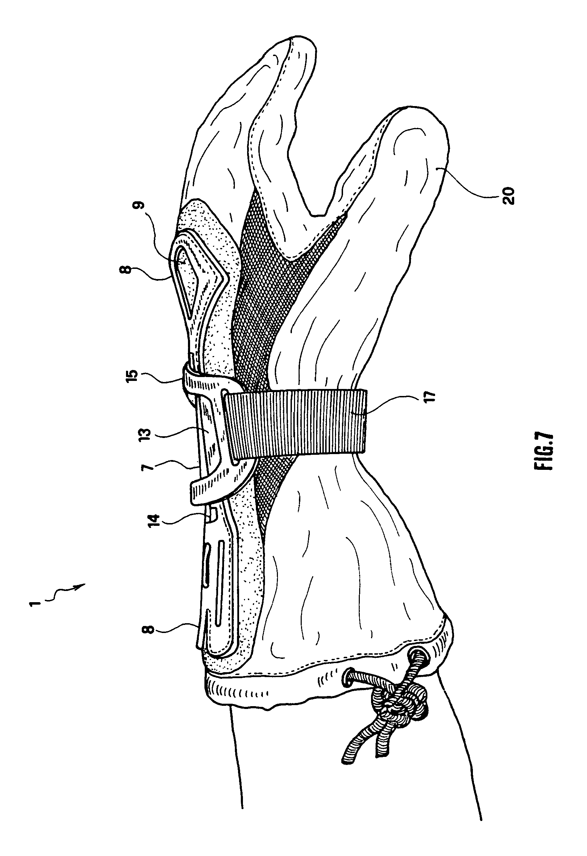

With reference to Figures 6 and 7, hereinafter a second embodiment of the orthosis 1 will be described, adopting the same number references for alike or equivalent components.

With respect to the preceding embodiment, here the means for wearing the orthosis on comprises a glove 20 on the back of which there are fixed the absorbing layer 12 (optionally incorporated in the thickness of the glove), the longitudinal protection elements 7 with the guide 13 and the shield-shaped element 15.

Otherwise, all is unchanged apart from the lack of the first means for fastening, redundant here since the glove already foresees to a correct positioning of the entire orthosis 1.

Moreover, it is proposed that the shape of the glove could adapt to the different sports activities, or even working activities, for which it is intended. It could also comprise additional protections, e.g. onto the palm for inline skating practice.

To the abovedescribed embodiments of orthosis for the protection of the wrist a person skilled in the art, in order to satisfy further and contingent needs, may effect several further modifications and variants, all however comprised within the protection scope of the present invention, as defined by the annexed claims.

## Claims

1. An orthosis for the protection of the wrist (1), particularly for use in sports, of the kind comprising stiff or semiflexible protection elements, at least partially capable of absorbing a shock, arranged at the wrist joint, comprising:
* means (2; 20) for wearing the orthosis on the wrist region, comprising a portion of the back of the hand, the wrist joint and a portion of forearm adjacent to the wrist;
* at least one longitudinal protection and stiffening element (7), associated to said means for wearing the orthosis on so as to extend over the entire wrist region;
**characterised in that**
the orthosis further comprises:
* a guide (13) longitudinally arranged at said at least one longitudinal protection element (7), astride of the wrist joint;
* a shield-shaped protection element (15) slidably associated to said guide (13); and
* means (17) for fastening said shield-shaped protection element (15) in a desired position.

2. The orthosis (1) according to claim 1, wherein said means for wearing on comprises a sleeve-shaped wrist brace (2).

3. The orthosis (1) according to claim 2, wherein the wrist brace (2) is made of breathable fabric.

4. The orthosis (1) according to claim 1, wherein said means for wearing on comprises a glove (20).

5. The orthosis (1) according to claim 1, comprising longitudinal protection and stiffening elements (7), extending over the entire wrist region.

6. The orthosis (1) according to claim 5, wherein said longitudinal protection elements (7) have, onto their ends, widened portions (8).

7. The orthosis (1) according to claim 6, wherein said widened portions (8) are provided with notches (9).

8. The orthosis (1) according to claim 5, wherein the longitudinal protection elements (7) are united by longitudinal junctions (10) made of a softer material.

9. The orthosis (1) according to claim 1, comprising means for fastening to the wrist region.

10. The orthosis (1) according to claim 9, wherein said means for fastening is implemented by straps (11) provided with Velcro^{®} at their ends in order to adjust its tensioning, or with analogous tensioning adjustment systems.

11. The orthosis (1) according to claim 1, wherein, between the means (2; 20) for wearing on and said at least one longitudinal protection element (7) there is arranged an absorbing layer (12), of flexible material, having the purpose of absorbing shocks, of a shape such as to cover substantially the entire wrist region.

12. The orthosis (1) according to claim 5, wherein between the two longitudinal protection elements (7) there is located said guide (13)

13. The orthosis (1) according to claim 1, wherein the guide (13), at one end thereof, has a transverse through recess (14) and wherein, onto its bottom face, the shield-shaped protection element (15) comprises a pair of projections (16) for sliding, shaped so as to be capable of going through said transverse recess (14) and inserting below the guide (13), with the stem of each projection (16) going through the port of the guide (13).

14. The orthosis (1) according to claim 1, wherein the shield-shaped protection element (15) is substantially shaped like a quadrilateral frame with slightly curved sides, in order to extend and entirely cover the wrist joint.

15. The orthosis (1) according to claim 1, wherein the means for fastening the shield-shaped protection element (15) are implemented by straps (17) provided with Velcro^{®} at their ends in order to adjust its tensioning, or with analogous tensioning adjustment systems.

16. Wrist braces (2), wherein there are incorporated respective orthoses (1) for the protection of the wrist according to one of the claims 1 to 15.

17. Gloves (20), wherein there are incorporated respective orthoses (1) for the protection of the wrist according to one of the claims 1 to 15.

## Patentansprüche

1. Orthese für den Schutz des Handgelenks (1), insbesondere zur Verwendung im Sport, in der Art mit steifen oder semiflexiblen Schutzelementen, wenigstens teilweise fähig, Stöße zur absorbieren, angeordnet am Handgelenk, mit:
* einem Mittel (2; 20) zum Tragen der Orthese auf der Handgelenksregion, mit einem Bereich des Handrückens, des Handgelenks und einem Bereich des an das Handgelenk angrenzenden Unterarms;
* wenigstens einem längsgerichteten Schutz- und Versteifungselement (7), das mit dem Mittel zum Tragen der Orthese so verbunden ist, das sich dieses über die gesamte Handgelenksregion erstreckt;
**dadurch gekennzeichnet, dass**
die Orthese ferner umfasst:
* eine Führung (13), die an dem wenigstens einen längsgerichteten Schutzelement (7) in Längsrichtung rittlings des Handgelenks angeordnet ist;
* ein schirmförmiges Schutzelement (15), das mit der Führung (13) gleitfähig verbunden ist; und
* Mitteln (17) zum Befestigen des schirmförmigen Schutzelements (15) in einer gewünschten Position.

2. Orthese (1) nach Anspruch 1, in welcher das Mittel zum Tragen eine schlauchförmige Handgelenksstütze (2) umfasst.

3. Orthese (1) nach Anspruch 2, in welcher die Handgelenksstütze (2) hergestellt ist aus einem atmungsfähigen Textilstoff.

4. Orthese (1) nach Anspruch 1, in welcher das Mittel zum Tragen einen Handschuh (20) umfasst.

5. Orthese (1) nach Anspruch 1, mit längsgerichteten Schutz- und Versteifungselementen (7), die sich über die gesamte Handgelenksregion erstrecken.

6. Orthese (1) nach Anspruch 5, in welcher die längsgerichteten Schutzelemente (7) auf ihren Enden geweitete Bereiche (8) haben.

7. Orthese (1) nach Anspruch 6, in welcher die geweiteten Bereiche (8) mit Aussparungen (9) versehen sind.

8. Orthese (1) nach Anspruch 5, in welcher die längsgerichteten Schutzelemente (7) durch Längsverbindungsteile (10) aus weicherem Material verbunden sind.

9. Orthese (1) nach Anspruch 1, mit Mitteln zum Befestigen an der Handgelenksregion.

10. Orthese (1) nach Anspruch 9, in welcher die Mittel zum Befestigen durch Bänder (11) hergestellt sind, die mit Velcro® an ihren Enden oder mit analogen Spannungs-Einstellsystemen versehen sind, um ihre Spannung einzustellen,.

11. Orthese (1) nach Anspruch 1, in welcher zwischen den Mitteln (2; 20) zum Tragen und dem wenigstens einen längsgerichteten Schutzelement (7) eine Absorptionsschicht (12) aus flexiblem Material angeordnet ist, die den Zweck zum Absorbieren von Stößen hat, und zwar in einer Form, dass diese im Wesentlichen die gesamten Handgelenksregion überdeckt.

12. Orthese (1) nach Anspruch 5, in welcher zwischen den zwei längsgerichteten Schutzelementen (7) die Führung (13) angeordnet ist.

13. Orthese (1) nach Anspruch 1, in welcher die Führung (13) an ihrem einen Ende eine quer verlaufende Durchgangsöffnung (14) hat, und in welcher auf ihrer Bodenseite das schirmförmige Schutzelement (15) ein Paar Vorsprünge (16) zum Gleiten umfasst, die so geformt sind, dass diese durch die Queröffnung (14) hindurchgehen und unter die Führung (13) eingeführt werden können, wobei der Schaft jedes Vorsprungs (16) durch den Anschluss der Führung (13) hindurchgeht.

14. Orthese (1) nach Anspruch 1, in welcher das schirmförmige Schutzelement (15) im Wesentlichen wie ein viereckiger Rahmen mit leicht geschwungenen Seiten hat, um sich über das Handgelenk zu erstrecken und dieses ganz abzudecken.

15. Orthese (1) nach Anspruch 1, in welcher die Mittel zum Befestigen des schirmförmigen Schutzelement (15) durch Bänder (17) verwirklicht sind, die mit Velcro® an ihren Enden oder mit analogen Spannungs-Einstellsystemen versehen sind, um ihre Spannung einzustellen,.

16. Handgelenkstützen (2), in welchen jeweilige Orthesen (1) für den Schutz des Handgelenks nach einem der Ansprüche 1 bis 15 eingebaut sind.

17. Handschuhe (20), in welchen jeweilige Orthesen (1) für den Schutz des Handgelenks nach einem der Ansprüche 1 bis 15 eingebaut sind.

## Revendications

1. Orthèse pour la protection du poignet (1), en particulier destinée à être utilisée pour le sport, du type comprenant des éléments de protection rigides ou semi-flexibles, au moins partiellement capable d'absorber un choc, agencés au niveau de l'articulation du poignet, comprenant :
des moyens (2 ; 20) pour porter l'orthèse sur la région du poignet, comprenant une partie du dos de la main, l'articulation du poignet et une partie de l'avant-bras adjacente au poignet ;
au moins un élément de protection et de renforcement longitudinal (7), associé auxdits moyens pour porter l'orthèse afin de s'étendre sur toute la région du poignet ;
**caractérisée en ce que** l'orthèse comprend en outre :
un guide (13) agencé longitudinalement au niveau dudit au moins un élément de protection longitudinal (7), à cheval sur l'articulation du poignet ;
un élément de protection en forme de bouclier (15) associé de manière coulissante audit guide (13) ; et
des moyens (17) pour fixer ledit élément de protection en forme de bouclier (15) dans une position souhaitée.

2. Orthèse (1) selon la revendication 1, dans laquelle lesdits moyens pour porter l'orthèse comprennent une attache de poignet en forme de manchon (2).

3. Orthèse (1) selon la revendication 2, dans laquelle l'attache de poignet (2) est réalisée avec un tissu perméable à l'air.

4. Orthèse (1) selon la revendication 1, dans laquelle lesdits moyens pour porter l'orthèse comprennent un gant (20).

5. Orthèse (1) selon la revendication 1, comprenant des éléments de protection et de renforcement longitudinaux (7) s'étendant sur toute la région du poignet.

6. Orthèse (1) selon la revendication 5, dans laquelle lesdits éléments de protection longitudinaux (7) ont des parties élargies (8), sur leurs extrémités.

7. Orthèse (1) selon la revendication 6, dans laquelle lesdites parties élargies (8) sont prévues avec des encoches (9).

8. Orthèse (1) selon la revendication 5, dans laquelle les éléments de protection longitudinaux (7) sont réunis par des jonctions longitudinales (10) réalisées avec un matériau plus souple.

9. Orthèse (1) selon la revendication 1, comprenant des moyens pour fixer la région de poignet.

10. Orthèse (1) selon la revendication 9, dans laquelle lesdits moyens de fixation sont mis en oeuvre par des sangles (11) dotées de Velcro^{®} au niveau de leurs extrémités afin d'ajuster sa tension ou avec des systèmes d'ajustement de tension analogues.

11. Orthèse (1) selon la revendication 1, dans laquelle, entre les moyens (2 ; 20) pour porter l'orthèse et ledit au moins un élément de protection longitudinal (7), on agence une couche absorbante (12) réalisée avec un matériau flexible, ayant pour but d'absorber les chocs, d'une forme telle qu'elle recouvre sensiblement toute la région de poignet.

12. Orthèse (1) selon la revendication 5, dans laquelle on positionne ledit guide (13) entre les deux éléments de protection longitudinaux (7).

13. Orthèse (1) selon la revendication 1, dans laquelle le guide (13), au niveau de son extrémité, a un évidement de passage transversal (14) et dans laquelle, sur sa face inférieure, l'élément de protection en forme de bouclier (15) comprend une paire de saillies (16) pour coulisser, formées afin de pouvoir traverser ledit évidement transversal (14) et être insérées au-dessous du guide (13), avec la tige de chaque protection (16) qui traverse l'orifice du guide (13).

14. Orthèse (1) selon la revendication 1, dans laquelle l'élément de protection en forme de bouclier (15) est formé de manière sensiblement identique à un châssis quadrilatère avec des côtés légèrement incurvés, afin de s'étendre et de recouvrir complètement l'articulation du poignet.

15. Orthèse (1) selon la revendication 1, dans laquelle les moyens pour fixer l'élément de protection en forme de bouclier (15) sont mis en oeuvre par des sangles (17) dotées de Velcro^{®} au niveau de leurs extrémités afin d'ajuster sa tension, ou avec des systèmes d'ajustement de tension analogues.

16. Attaches de poignet (2), dans lesquelles on incorpore des orthèses (1) respectives pour la protection du poignet selon l'une quelconque des revendications 1 à 15.

17. Gants (20), dans lesquels on incorpore des orthèses (1) respectives pour la protection du poignet selon l'une quelconque des revendications 1 à 15.
